# EUROPEAN PATENT APPLICATION

(11) **EP 4 421 169 A1**
(43) Date of publication of application: **28.08.2024**
(21) Application number: 22898488.6
(22) Date of filing: 16.11.2022
(51) Int. Cl.: C12N 15/10, C12Q 1/6806, C12Q 1/6844, C12Q 1/6851, C12Q 1/686, C12M 1/00, C12M 1/12

(54) **NUCLEIC ACID TEST METHOD AND TEST KIT**

(30) Priority: 25.11.2021 JP 2021191592
(71) Applicant: FUJIFILM Corporation, Tokyo 106-8620 (JP)
(72) Inventor: HAMA, Takeshi, Ashigarakami-gun, Kanagawa 258-8538 (JP)
(74) Representative: Frommberger, Moritz
(86) International application number: PCT/JP2022/042617
(87) International publication number: WO 2023/095704

(57) **Abstract**

The nucleic acid examination method includes an extraction step of extracting a nucleic acid from a specimen, a purification step of removing impurities in a liquid of any of a solution containing the specimen or a nucleic acid extraction liquid from which the nucleic acid has been extracted, at any stage before, during, or after the extraction step, an amplification step of amplifying a base sequence in the nucleic acid extraction liquid, a detection step of detecting presence or absence of the base sequence after the amplification step. The extraction step is a step of bringing the specimen into contact with an extraction reagent containing a nonionic surfactant, and the purification step includes an adsorption step of bringing a liquid into contact with a zeolite having any crystal structure of a ferrierite, a mordenite, an L-type, or a Y-type, to adsorb impurities in the liquid to the zeolite.

## Description

### BACKGROUND OF THE INVENTION

### 1. Field of the Invention

The present disclosure relates to a nucleic acid examination method and an examination kit.

### 2. Description of the Related Art

In techniques for gene diagnosis, it is known an examination method in which a trace amount of a nucleic acid contained in a specimen collected from a living body is subjected to an amplification treatment for amplification of a base sequence as an examination target, and an examination is carried out to examine whether or not a nucleic acid including the base sequence as an examination target is present in the specimen. Examples of the amplification method include a polymerase chain reaction (PCR) method and a loop-mediated isothermal amplification (LAMP) method.

In the above-described examination method, for example, first, an extraction step of charging a specimen into an extraction reagent to extract a nucleic acid from the specimen is carried out in order to extract the nucleic acid from the collected specimen. Since the nucleic acid is present in cells, it is necessary to lyse the cell to elute the nucleic acid. The extraction step is a step of lysing cells with an extraction reagent to elute the nucleic acid. Thereafter, a purification step of removing impurities from a nucleic acid extraction liquid obtained by extracting a nucleic acid from a specimen is carried out. The impurities include impurities derived from a living body, which are contained in a specimen, and impurities such as proteins or polysaccharides, which are eluted together with the nucleic acid during the extraction of the nucleic acid. Such impurities are removed because they are factors that inhibit the amplification of the nucleic acid. The nucleic acid extraction liquid that has undergone the purification step is mixed with an amplification reagent for amplifying a base sequence as an examination target, and an amplification step of carrying out an amplification treatment by a PCR method, a LAMP method, or the like is carried out. Thereafter, a signal from a probe attached to the base sequence as an examination target is detected to examine whether or not the base sequence as an examination target is included. Here, the nucleic acid is a general term for deoxyribonucleic acid (DNA) and ribonucleic acid (RNA).

WO2009/060847A describes that in an extraction step, an extraction reagent containing an anionic surfactant or an alkali is used as an extraction reagent, that in a purification step, a nucleic acid extraction liquid is brought into contact with zeolite to remove impurities by an adsorption action of the zeolite, and that in an amplification step, the LAMP method is used. A purification step using zeolite is preferable because it is simple as compared with, for example, a method of separating impurities and a nucleic acid using magnetic particles.

### SUMMARY OF THE INVENTION

However, in a case where the method described in WO2009/060847A was applied to the PCR method instead of the LAMP method, there was a case where the amplification was insufficient. Since the amplification was insufficient, there was a case where the examination accuracy was lower in a case where a trace amount of the nucleic acid in the specimen was used.

The present disclosure has been made in consideration of the above circumstances, and an object of the present disclosure is to provide a nucleic acid examination method and an examination kit, which make it possible to realize a nucleic acid examination with high examination accuracy as compared with those in the related art, where the nucleic acid examination is not limited by a specific amplification method.

The nucleic acid examination method according to the present disclosure is a nucleic acid examination method for examining whether or not a base sequence as an examination target is present in a nucleic acid contained in a specimen collected from a living body, the nucleic acid examination method including:
an extraction step of extracting the nucleic acid from the specimen;
a purification step of removing impurities in a liquid of any of a solution containing the specimen or a nucleic acid extraction liquid from which the nucleic acid has been extracted, at any stage before, during, or after the extraction step;
an amplification step of amplifying the base sequence in the nucleic acid extraction liquid; and
a detection step of detecting presence or absence of the base sequence after the amplification step,
in which the extraction step is a step of bringing the specimen into contact with an extraction reagent containing a nonionic surfactant, and
the purification step includes an adsorption step of bringing the liquid into contact with a zeolite having any crystal structure of a ferrierite, a mordenite, an L-type, or a Y-type, to adsorb the impurities in the liquid to the zeolite.

In the nucleic acid examination method according to the present disclosure, it is preferable that the purification step includes, after the adsorption step, a filtration step of filtering the liquid to remove the zeolite from the liquid.

In the nucleic acid examination method according to the present disclosure, it is preferable that in the extraction step, the zeolite is added into the extraction reagent to carry out the extraction step and the purification step in parallel.

In the nucleic acid examination method according to the present disclosure, the purification step may be carried out after the extraction step.

In the nucleic acid examination method according to the present disclosure, it is preferable that the nonionic surfactant includes at least one of polyethylene oxide, glucamine, or betaine.

In the nucleic acid examination method according to the present disclosure, it is preferable that a crystal structure of the zeolite is any one of a ferrierite, an L-type, or a Y-type.

In the nucleic acid examination method according to the present disclosure, it is preferable that a cation of the zeolite is any one of Na⁺, K⁺, or H⁺.

In the nucleic acid examination method according to the present disclosure, it is preferable that a micropore diameter of the zeolite is more than 0.7 nm and less than 1 nm.

In the nucleic acid examination method according to the present disclosure, it is preferable that a ratio Si/Al of silicon to aluminum in the zeolite is 5 or more and 18 or less.

In the nucleic acid examination method according to the present disclosure, it is preferable that the amplification step is an amplification step by a polymerase chain reaction.

The examination kit according to the present disclosure is an examination kit that is used for examining whether or not a base sequence as an examination target is present in a nucleic acid contained in a specimen collected from a living body, the examination kit including:
an extraction container accommodating an extraction reagent that contains a nonionic surfactant; and
a cartridge including;
   a plurality of accommodating parts that includes an amplification reagent accommodating part accommodating an amplification reagent that is used for amplification of the nucleic acid, and
   a plurality of flow channels that connect the plurality of accommodating parts to each other,
in which a zeolite having any crystal structure of a ferrierite, a mordenite, an L-type, or a Y-type is provided in at least one of the extraction container or the cartridge or in a form in which the zeolite is capable of being charged into the extraction container.

The examination kit according to the present disclosure preferably includes a filter for filtering a nucleic acid extraction liquid, the filter having meshes smaller than the zeolite.

The filter may be provided in the cartridge or may be provided separately from the cartridge.

The filter may be provided in a path through which a liquid in the extraction container is charged into the cartridge or at an inlet of the cartridge for the liquid.

The cartridge may accommodate the zeolite in one accommodating part that is different from the amplification reagent accommodating part among the plurality of accommodating parts and may include a flow channel in a flow channel from the accommodating part in which the zeolite is accommodated to the amplification reagent accommodating part.

In the examination kit according to the present disclosure, the cartridge includes an inlet into which a liquid is to be charged; an attachable and detachable lid portion that covers the inlet; a first accommodating part that is capable of accommodating a liquid and is provided at a position facing the inlet; a second accommodating part that is the amplification reagent accommodating part accommodating the amplification reagent; a first flow channel that connects the first accommodating part to the second accommodating part; a first cylinder in which one end is connected to the first accommodating part through a second flow channel and another end is open to an outside; a second cylinder in which one end is connected to the second accommodating part through a third flow channel and another end is open to the outside; a first plug that is provided to be movable in the first cylinder; and a second plug that is provided to be movable in the second cylinder, wherein the cartridge may be configured such that an inner space including the first accommodating part, the second accommodating part, the first flow channel, the second flow channel, and the third flow channel is capable of being pressurized by pressing the first plug and the second plug to be moved from the outside, each of the first accommodating part and the second accommodating part may be one of the plurality of accommodating parts, and each of the first flow channel, the second flow channel, and the third flow channel may be one of the plurality of flow channels.

According to the nucleic acid examination method and the examination kit according to the present disclosure, it is possible to realize a nucleic acid examination with high examination accuracy as compared with those in the related art, where the nucleic acid examination is not limited by a specific amplification method.

### BRIEF DESCRIPTION OF THE DRAWINGS

Fig. 1 is a view showing a schematic configuration of an examination kit 1 according to one embodiment.
Fig. 2 is a plan view of a cartridge 10 shown in Fig. 1.
Fig. 3A shows a cross section taken along a line 3A-3A of Fig. 2, Fig. 3B shows a cross section taken along a line 3B-3B of Fig. 2, and Fig. 3C shows a cross section taken along a line 3C-3C of Fig. 2.
Fig. 4 is a view showing a schematic configuration of an examination device 100 according to one embodiment.
Fig. 5A is a plan view showing a positional relationship between the cartridge 10 and a pressing machine 108 in the examination device 100, and Fig. 5B is a cross-sectional view taken along a line 5B-5B of Fig. 5A.
Fig. 6 is a flowchart for describing a flow of a nucleic acid examination method.
Fig. 7 is a view for describing an extraction step and a purification step in a nucleic acid examination method according to one embodiment.
Fig. 8 is a view for describing an extraction step and a purification step in a nucleic acid examination method of a modification example 1.
Fig. 9 is a flowchart for describing a flow of a nucleic acid examination method of a modification example 2.
Fig. 10 is a plan view of a cartridge 11 of a design modification example.

### DESCRIPTION OF THE PREFERRED EMBODIMENTS

Hereinafter, embodiments of the nucleic acid examination method and the examination kit according to the present disclosure will be described with reference to the drawings. The nucleic acid examination method according to the present disclosure a method of examining whether or not a base sequence as an examination target is present in a nucleic acid contained in a specimen collected from a living body. First, a description will be made for one example of an examination kit and an examination device, which are used in a nucleic acid examination method according to one embodiment, and then, a description will be made for a nucleic acid examination method according to one embodiment.

### [Examination kit]

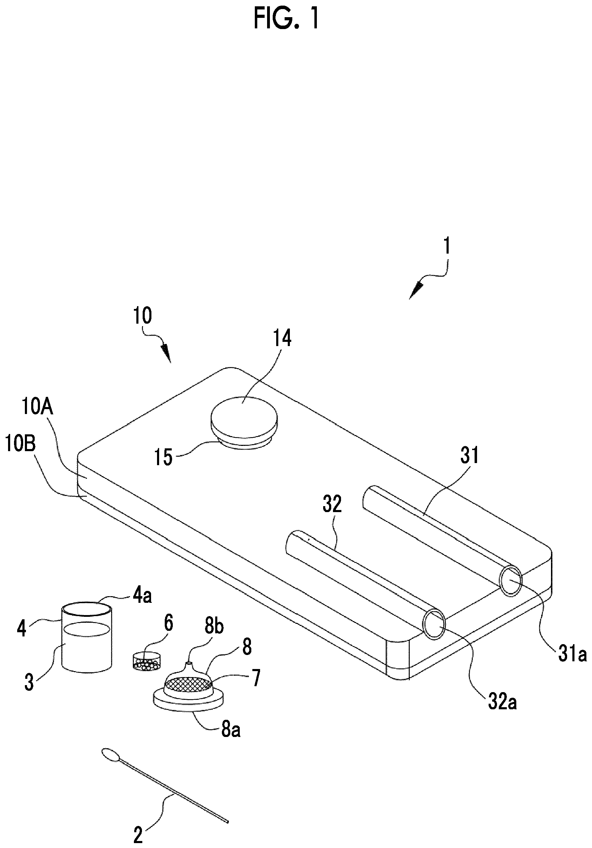

Fig. 1 is a view showing a schematic configuration of an examination kit 1, which is used in a nucleic acid examination method according to one embodiment.

The examination kit 1 is an examination kit that is used for examining whether or not a base sequence as an examination target is present in a nucleic acid contained in a specimen collected from a living body. Specifically, it is used for examining whether or not a living body has an infectious disease due to infection with an influenza virus or a novel coronavirus.

The examination kit 1 includes a specimen collection tool 2, an extraction container 4 accommodating an extraction reagent 3, a zeolite 6, a funnel 8 including a filtration filter 7, and a cartridge 10.

The specimen collected from a living body is a biological specimen that is collected from a living body such as an individual of a target to be examined, more specifically, an animal including a human. It includes, for example, blood, serum, blood plasma, spinal fluid, tear fluid, sweat, urine, feces, pus, nasal discharge, nasal swab, pharynx swab, nasal aspirate, or sputum, and further, an organ, a tissue, a mucous membrane, a skin, or the like. It is noted that the solution containing a specimen may contain a specimen and a solvent; however, it may be the specimen itself in a case where the specimen is in a liquid state. In addition, a liquid transferred to a transport medium may be used as a specimen.

The specimen collection tool 2 is a tool for collecting a specimen, and a swab for collecting the nasal swab is used in the present example. As the specimen collection tool 2, an appropriate collection tool can be provided depending on the specimen.

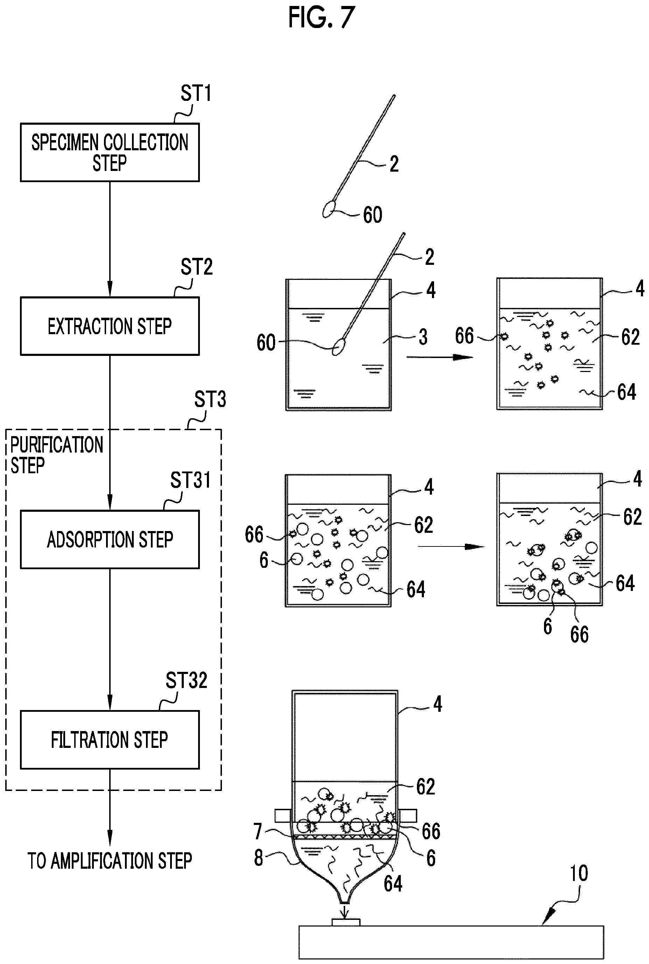

The extraction container 4 accommodates the extraction reagent 3 for extracting a nucleic acid from a specimen. In a case where the extraction reagent 3 is brought into contact with a specimen, a nucleic acid is extracted from the specimen. For example, the nucleic acid is extracted by bringing the extraction reagent 3 into contact with a specimen in the extraction container 4. The extraction reagent 3 containing the extracted nucleic acid is hereinafter referred to as a nucleic acid extraction liquid 62 (see Fig. 7). The extraction reagent 3 according to the present disclosure contains a nonionic surfactant as a surfactant but does not contain an anionic surfactant.

It is preferable that the nonionic surfactant includes at least one of polyethylene oxide, glucamine, or betaine. It is noted that the nonionic surfactant may include two or more among polyethylene oxide, glucamine, and betaine. The concentration of the nonionic surfactant in the extraction reagent is, for example, about 0.1 vol% to 5 vol%, and it is preferably 0.1 vol% to 2 vol%.

The zeolite 6 is used to adsorb impurities. The zeolite 6 is a general term for a porous crystalline alumino silicate, and it is generally represented by MeO·Al2O₃·mSiO₂·nH₂O. Me represents a monovalent or divalent cation (that is, a positive ion), and m represents a ration of silicon to aluminum (that is, m = Si/Al). In the present embodiment, a zeolite having any crystal structure of a ferrierite, a mordenite, an L-type, or a Y-type is used as the zeolite 6. The crystal structure of the zeolite 6 is more preferably a ferrierite, an L-type, or a Y-type. The zeolite 6 has, for example, a size of submicron order.

In the zeolite 6, the cation Me may be any monovalent or divalent cation, and for example, a sodium ion (Na⁺), a potassium ion (K⁺), a hydrogen ion (H⁺), and ammonium (NH₄⁺) can be adopted. The cation Me is preferably any one of Na⁺, K⁺, or H⁺.

A micropore diameter of the zeolite 6 is generally 0.2 nm to 1 nm; however, it is preferably larger than 0.7 nm and smaller than 1 nm.

A ratio m of silicon to aluminum in the zeolite 6 is preferably 5 or more and 18 or less.

The impurities include impurities derived from a living body, which are contained in a specimen during the collection of the specimen from the living body, impurities such as a cell wall, which are generated during the extraction of the nucleic acid, and the like. The impurities include a protein that is secreted in the nasal cavity, and the like, for example, in a case where the specimen is the nasal swab. Some of the impurities inhibit nucleic acid amplification, and the zeolite 6 adsorbs the impurities that inhibit the nucleic acid amplification.

The filtration filter 7 is a filter that separates the zeolite 6 from a solution to which the zeolite 6 has been added, where the filtration filter 7 is a filter having meshes smaller than the zeolite 6. Here, the phrase "a filter having meshes smaller than the zeolite 6" means that a filter has a performance of allowing a nucleic acid extraction liquid described later to pass through without allowing the zeolite 6 to permeate through and specifically means a filter having a pore diameter smaller than the particle shape of the zeolite 6.

Examples of the material of the filtration filter 7 include cellulose acetate, nitrocellulose, a cellulose mixed ester, polyether sulfone, polytetrafluoroethylene (PTFE), poly vinylidene difluoride (PVDF), nylon, polyamide, polycarbonate, polypropylene, polyvinyl chloride, and glass fiber. The filtration filter 7 is, for example, a microfiltration membrane having a mesh size (pore diameter) of approximately 50 nm to 10 µm. The microfiltration membrane includes a membrane filter that captures a tangible substance on the surface and a prefilter (for example, a filter paper or a depth filter) that captures a tangible substance inside the filter, and it is appropriately selected according to the application.

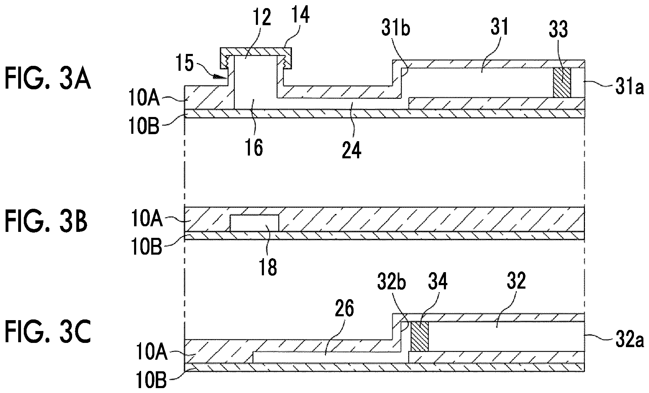

The funnel 8 is used to charge the nucleic acid extraction liquid 62 (see Fig. 7) in the extraction container 4 into the cartridge 10. The funnel 8 includes an injection opening 8a and a discharge opening 8b. The funnel 8 has a shape in which the opening diameter gradually decreases from the injection opening 8a toward the discharge opening 8b. The funnel 8 is attached to the extraction container 4 by fitting the opening portion 4a of the extraction container 4 to the injection opening 8a. In addition, in a case where the nucleic acid extraction liquid 62 in the extraction container 4 is charged into the cartridge 10, the injection opening 8a of the funnel 8 attached to the extraction container 4 is inserted into an inlet 12 of the cartridge 10 (see Fig. 3).

In the present example, the filtration filter 7 is provided between the injection opening 8a of the funnel 8 and the discharge opening 8b thereof. As a result, the nucleic acid extraction liquid 62 injected from the injection opening 8a passes through the filtration filter 7 and then is discharged from the discharge opening 8b. As described above, in the present example, the filtration filter 7 is provided separately from the cartridge 10.

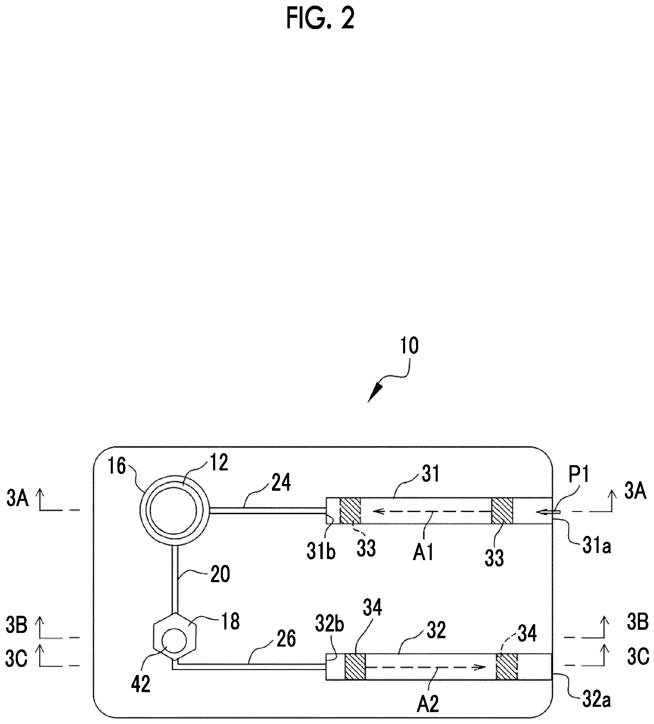

The cartridge 10 will be described in detail. Fig. 2 is a plan view of the cartridge 10. Fig. 3A of Fig. 3 shows a cross section taken along a line 3A-3A of Fig. 2, Fig. 3B shows a cross section taken along a line 3B-3B of Fig. 2, and Fig. 3C shows a cross section taken along a line 3C-3C of Fig. 2. The cartridge 10 has, for example, a planar size of about a credit card size and a thickness of about 1 cm.

As shown in Fig. 1 and Fig. 3, the cartridge 10 is composed of a body member 10A, in which a recessed part that constitutes a part of a flow channel structure including a flow channel and a accommodating part and including a hole portion have been formed, and a bottom member 10B that constitutes a bottom surface of the flow channel.

For the body member 10A, any publicly known resin molding plastic material can be used without particular limitation; however, it is preferably polycarbonate, polypropylene, cycloolefin, or a silicone resin from the viewpoint of heat resistance and transparency.

The bottom member 10B is formed from, for example, a thin plate or a film. For the bottom member 10B, any publicly known resin molding plastic material can be used without particular limitation; however, it is preferably the same material as the body member 10A from the viewpoint of adhesiveness to the body member 10A.

As shown in Fig. 1 to Fig. 3, the cartridge 10 includes an inlet 12, a lid portion 14, a first accommodating part 16, a second accommodating part 18, a first flow channel 20, a first cylinder 31, a second cylinder 32, a first plug 33, and a second plug 34. In addition, the cartridge 10 further includes a second flow channel 24 and a third flow channel 26.

The inlet 12 is an opening for charging the nucleic acid extraction liquid 62 (see Fig. 7) which is an example of a liquid. The lid portion 14 is a lid portion that covers the inlet 12 and is attachable and detachable to the opening of the inlet 12. In the present example, the lid portion 14 is formed to be screwable with a tubular portion 15 that forms the inlet 12.

It is noted that, hereinafter, the surface on which the inlet 12 is provided is referred to as an upper surface of the cartridge 10, and the bottom member 10B side is referred to as a lower surface of the cartridge 10. Here, the upper surface of the body member 10A is the same as the upper surface of the cartridge 10, the lower surface of the body member 10A is a surface in contact with the upper surface of the bottom member 10B, and the lower surface of the bottom member 10B is the same as the lower surface of the cartridge 10.

The first accommodating part 16 is provided at a position facing the inlet 12, and it accommodates the nucleic acid extraction liquid 62 that has been dropped from the inlet 12. The shape of the first accommodating part 16 is not particularly limited, and any shape such as a columnar shape, a conical shape, or a truncated conical shape can selected as the shape of the first accommodating part 16.

In the present cartridge 10, the inlet 12 is composed of the opening of the tubular portion 15 that is formed to penetrate the body member 10A in a thickness direction and to protrude from the surface of the body member 10A, and the first accommodating part 16 is formed from an inner side portion of the tubular portion 15 in the body member 10A and the bottom member 10B.

The second accommodating part 18 is a accommodating part that is capable of accommodating a liquid, and it functions as a reaction section in which the nucleic acid extraction liquid 62 (see Fig. 7) is reacted with a reagent 42. The reagent 42 includes an amplification reagent for amplifying a base sequence as an examination target, a probe for detecting a base sequence as an examination target, and the like. The second accommodating part 18 is formed from a recessed part, which is provided on the lower surface of the body member 10A, and the bottom member 10B.

In the present example, the reagent 42 is accommodated in the second accommodating part 18 in advance. However, it suffices that the reagent 42 is provided between the first accommodating part 16 and the second accommodating part 18, and the reagent 42 does not necessarily need to be accommodated in the second accommodating part 18. In the present example, the second accommodating part 18 constitutes an amplification reagent accommodating part. Examples of the amplification reagent include a primer, a polymerase, a substrate such as dNTP, and a salt. Further, an additive such as a reducing agent, a buffer, or the like may be included. In a case where the nucleic acid to be amplified is an RNA, a reverse transcription primer and a reverse transcriptase may be further included. The reagent 42 is appropriately selected according to an amplification method and a detection method. For example, in a case where the detection of the base sequence as an examination target is carried out according to a fluorescence method, the reagent 42 can include an amplification reagent and a fluorescence probe. The form of a reagent to be enclosed is not particularly limited, and any liquid or solid reagent can also be used. For example, a powdery reagent prepared by lyophilizing a liquid reagent, or a reagent molded into a pellet shape or a granule shape may be enclosed.

The first flow channel 20 connects the first accommodating part 16 to the second accommodating part 18. The nucleic acid extraction liquid 62 that has been charged into the first accommodating part 16 is fed to the second accommodating part 18 through the first flow channel 20. The first flow channel 20 is formed from a linear recessed part that extends from the first accommodating part 16 to the second accommodating part 18, on the lower surface of the body member 10A, and the upper surface of the bottom member 10B. It is noted that similarly, each of the second flow channel 24 and the third flow channel 26 is also formed from a linear recessed part that is formed on the lower surface of the body member 10A, and the upper surface of the bottom member 10B (see Fig. 3).

The first cylinder 31 is connected to the first accommodating part 16 through the second flow channel 24, where the first cylinder 31 is provided such that one end 31b communicates with the second flow channel 24, and the other end 31a is open to the outside.

The second cylinder 32 is connected to the second accommodating part 18 through the third flow channel 26, where the second cylinder 32 is provided such that one end 32b communicates with the third flow channel 26, and the other end 32a is open to the outside.

The first cylinder 31 and the second cylinder 32 are tubular portions formed in a plane direction of the body member 10A and are formed from an end of the body member 10A toward the inside thereof.

The first plug 33 is provided to be movable in the first cylinder 31. The second plug 34 is provided to be movable in the second cylinder 32. The first plug 33 and the second plug 34 are, for example, rubber plugs, and have functions of blocking outside air in the first cylinder 31 and the second cylinder 32, respectively.

A first pressing unit of a pressing machine to be described later can be inserted into the first cylinder 31 from the other end 31a, which is open to the outside, and thus the first plug 33 can be pressed toward an inner space in the first cylinder 31 by the first pressing unit. Similarly, a second pressing unit of the pressing machine to be described later can be inserted into the second cylinder 32 from the other end 32a, which is open to the outside, and thus the second plug 34 can be pressed toward an inner space in the second cylinder 32 by the second pressing unit. The inner space is under the atmospheric pressure in a state where the plug is not pressed by the pressing machine.

The cartridge 10 is configured such that the inner space including the first accommodating part 16, the second accommodating part 18, the first flow channel 20, the second flow channel 24, and the third flow channel 26 can be pressurized in a case where the first plug 33 and the second plug 34 are pressed and then moved from the outside. There is a possibility that gas in the nucleic acid extraction liquid 62 and the reagent 42 is generated as foam and inhibits the nucleic acid amplification. It is possible to suppress foaming and to suppress the inhibition of nucleic acid amplification, which is caused by foaming, by pressurizing the inner space in a case where the nucleic acid extraction liquid 62 is mixed with the reagent 42 in the second accommodating part 18 and liquid present in the second accommodating part 18 is then heated. For this reason, a nucleic acid amplification step in the second accommodating part 18 can be caused to proceed without being inhibited, and thus it is possible to improve examination accuracy without causing a delay in amplification time or insufficient amplification.

In a case where the nucleic acid extraction liquid 62 is accommodated in the first accommodating part 16, and the inlet 12 is closed by the lid portion 14, the inner space of the cartridge 10 is sealed by the first plug 33 and the second plug 34. As shown in Fig. 2, the first plug 33 is disposed closer to the other end 31a, which is open to the outside, than to the center of the first cylinder 31 in a length direction in an initial state before feeding a liquid. On the other hand, the second plug 34 is disposed closer to one end 32b than to the center of the second cylinder 32 in a length direction. In a case where the first plug 33 is pressed from the outside in the first cylinder 31 (arrow P1) and is moved toward the inner space as shown by a broken line arrow A1 as shown in Fig. 2 in this state, the inner space is pressurized. As a result, the second plug 34 positioned in the second cylinder 32 is interlocked and moved to be pushed out from the inner space to the outside as shown by a broken line arrow A2. Since the second plug 34 is moved while being interlocked with the movement of the first plug 33, the pressure of the inner space is adjusted and the nucleic acid extraction liquid 62 accommodated in the first accommodating part 16 can be fed to the second accommodating part 18 (arrow B). Since the second plug 34 is movable while being interlocked with the movement of the first plug 33, liquid can be fed with weak pressing. It is noted that the movement of the first plug 33 and the second plug 34 may be independently controlled to carry out liquid feeding.

### "Examination device"

Next, a description will be made for an example of an examination device 100 in which the above-described cartridge 10 is loaded, and an amplification step and a detection step, which will be described later, are carried out.

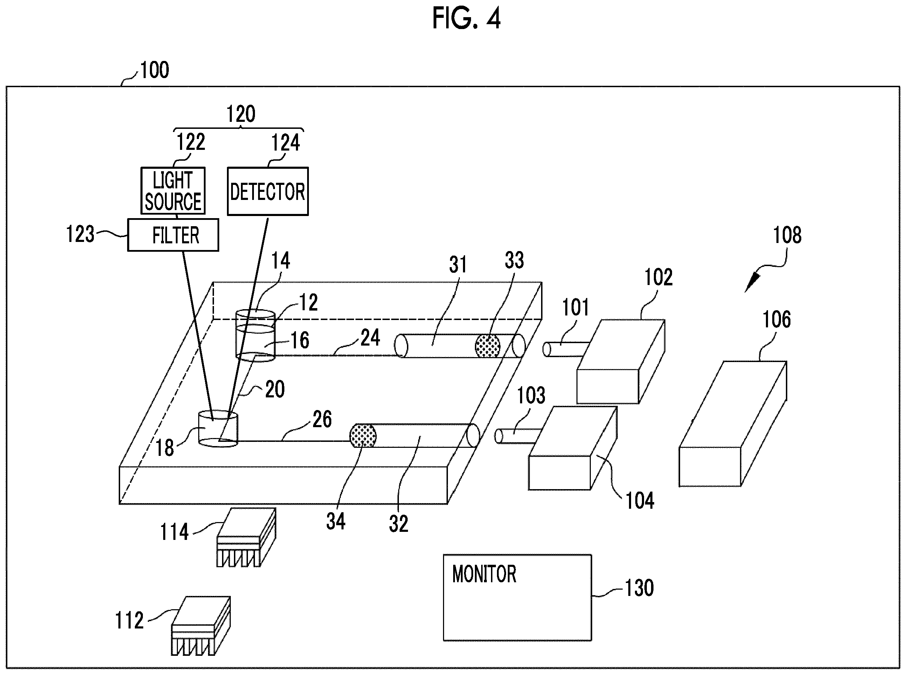

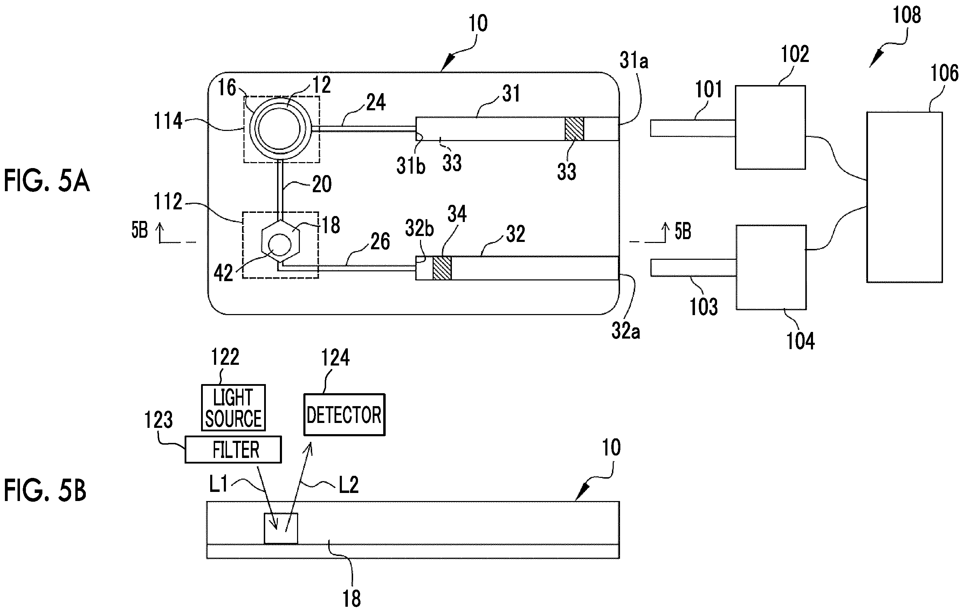

Fig. 4 is a view showing a schematic configuration of the examination device 100. The examination device 100 includes a pressing machine 108, a first heating unit 112, a second heating unit 114, a detection unit 120, and a monitor 130. In addition, the examination device 100 includes a accommodating part that accommodates the cartridge 10 attachably and detachably. Fig. 5A is a plan view showing a positional relationship between the cartridge 10 and a pressing machine 108 in the examination device 100. In addition, Fig. 5B is a cross-sectional view taken along a line 5B-5B of Fig. 5A, which shows a positional relationship between the cartridge 10 and the detection unit 120 in the examination device 100.

The pressing machine 108 includes a first pressing unit 102 that includes a first push rod 101, a second pressing unit 104 that includes a second push rod 103, and a pressing control unit 106 that controls the first pressing unit 102 and the second pressing unit 104. The first pressing unit 102 and the second pressing unit 104 can push in or pull out the first push rod 101 and the second push rod 103 with an actuator that uses a stepping motor, a solenoid, or the like. The actuator may be configured to use power such as pneumatic pressure.

The first pressing unit 102 is disposed at a position where the first push rod 101 can be inserted into the first cylinder 31 from the other end 31a open to the outside of the first cylinder 31 in a state where the cartridge 10 is installed. The first plug 33 can be pressed and then moved in the first cylinder 31 toward the inner space by the first push rod 101.

The second pressing unit 104 is disposed at a position where the second push rod 103 can be inserted into the second cylinder 32 from the other end 32a open to the outside of the second cylinder 32 in a state where the cartridge 10 is installed. The second plug 34 can be pressed and then moved in the second cylinder 32 toward the inner space by the second push rod 103.

The nucleic acid extraction liquid 62 is charged into the first accommodating part 16 of the cartridge 10, and then the lid portion 14 is closed to seal the inner space. In this way, the first plug 33 is pressed by the first pressing unit 102 in a state where the inner space is sealed, and then it is moved to the inner space, whereby the nucleic acid extraction liquid 62 accommodated in the first accommodating part 18 of the cartridge 10 can be fed to the second accommodating part 18.

Further, in a case where the first plug 33 is pressed by the first pressing unit 102 and the second plug 34 is pressed by the second pressing unit 104, the inner space of the cartridge 10 can be pressurized.

The first heating unit 112 is provided at a position that allows the first heating unit 112 to be in contact with the bottom surface of the second accommodating part 18 of the cartridge 10. The first heating unit 112 heats liquid accommodated in the second accommodating part 18. Here, the liquid accommodated in the second accommodating part 18 is a mixed liquid of the nucleic acid extraction liquid 62 and the reagent 42. The first heating unit 112 heats the mixed liquid of the nucleic acid extraction liquid 62 and the reagent 42 to facilitate the nucleic acid amplification.

The second heating unit 114 is provided at a position that allows the second heating unit 114 to be in contact with the bottom surface of the first accommodating part 16 of the cartridge 10. The second heating unit 114 heats liquid accommodated in the first accommodating part 16. Here, the liquid to be accommodated in the first accommodating part 16 is the nucleic acid extraction liquid 62. The second heating unit 114 heats the nucleic acid extraction liquid 62 for the pretreatment. The examination device 100 may not include the second heating unit 114 in a case where the heating of the nucleic acid extraction liquid 62 for pretreatment is not required.

The first heating unit 112 includes a Peltier element or the like and is configured to be capable of controlling a temperature, and it carries out a temperature cycle in the amplification step. On the other hand, the second heating unit 114 does not require the temperature cycle that is carried out by the first heating unit 112, and it is composed of, for example, a heater. A publicly known heating mechanism can be used as a heating mechanism to be used for each of the first heating unit 112 and the second heating unit 114, and the heating mechanism is not particularly limited.

The detection unit 120 detects whether or not an object to be detected is included in the nucleic acid extraction liquid 62 in the second accommodating part 18. The detection unit 120 includes an excitation light source 122, a wavelength selective filter 123, and a photodetector 124. The detection unit 120 is disposed above the second accommodating part 18 of the cartridge 10. The excitation light source 122 irradiates the inside of the second accommodating part 18 with excitation light L1 having a specific wavelength through the wavelength selective filter 123. The photodetector 124 detects fluorescence L2 that is excited by the excitation light L1 and is generated from a fluorescence probe. The excitation light L1 is selected according to an excitation wavelength of the fluorescence probe. In addition, a filter that adjusts intensity or an amount of light, a lens that is used to converge the excitation light L1 or to condense the fluorescence L2 generated from a detection probe to the photodetector 124, an optical system, or the like, may be included as necessary.

As the excitation light source 122, a light emitting diode (LED), a laser, or the like is used. The wavelength selective filter 123 is a filter that transmits only light having a wavelength corresponding to the excitation wavelength of the probe of the light emitted from the excitation light source 122. As the photodetector 124, for example, a photodiode or a photomultiplier tube, or an imaging device such as a camera is applied.

The monitor 130 is, for example, a touch panel display, and starts measurement or displays examination results in a case where a touch panel is operated.

### "Nucleic acid examination method"

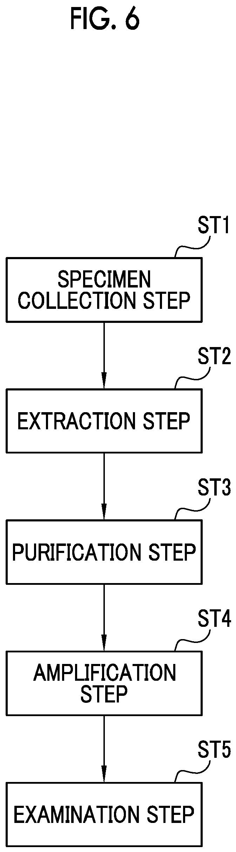

A nucleic acid examination method according to one embodiment of the present disclosure will be described. As shown in Fig. 6, the nucleic acid examination method according to one embodiment includes a specimen collection step ST1 of collecting a specimen, an extraction step ST2, a purification step ST3, an amplification step ST4, and an examination step ST5.

The specimen collection step ST1 is a step of collecting a specimen from a subject. It is noted that the specimen collection may be carried out separately from the series of the flow. The specimen may be collected by a collector different from the subject or may be collected by the subject himself/herself.

The extraction step ST2 is a step of extracting a nucleic acid from the specimen. Here, in the extraction step ST2, a specimen 60 is brought into contact with the extraction reagent 3 containing a nonionic surfactant.

The purification step ST3 is a step of removing impurities 66 (see Fig. 7) in any of the solution containing the specimen 60 or the nucleic acid extraction liquid 62 (see Fig. 6) from which the nucleic acid has been extracted. Specifically, a liquid of any of the solution containing the specimen 60 or the nucleic acid extraction liquid 62 is brought into contact with the zeolite 6 having any crystal structure of a ferrierite, a mordenite, an L-type, or a Y-type. The purification step includes an adsorption step of bringing the solution containing the specimen 60 or the nucleic acid extraction liquid 62 into contact with the zeolite 6, as described above, to adsorb the impurities 66 to the zeolite. Although the purification step ST3 is described as a post-step of the extraction step ST2 in Fig. 6, the purification step ST3 may be carried out at any stage before, during, or after the extraction step ST2.

The amplification step ST4 is a step of amplifying a base sequence as an examination target in the nucleic acid extraction liquid 62.

The examination step ST5 is a step of examining the presence or absence of the base sequence as an examination target after the amplification step ST4.

Hereinafter, a description will be made for a more specific nucleic acid examination method that uses the examination kit 1 and the examination device 100. In the present examination method, the specimen collection step ST1, the extraction step ST2, and the purification step ST3 are carried out outside the cartridge 10, and the amplification step ST4 and the examination step ST5 are carried out using the cartridge 10.

First, a procedure up to the purification step ST3 that is carried out outside the cartridge 10 will be described with reference to Fig. 7.

The specimen 60 is collected from a living body using the specimen collection tool 2 (the collection step ST1). Specifically, a specimen is collected from the nasal cavity, the pharynx, the inside of the oral cavity, or the affected part of the subject by using the specimen collection tool 2. Alternatively, a body fluid such as a washing solution from the nasal cavity, the pharynx, or the inside of the oral cavity, saliva, urine, or blood is collected as a specimen. In the present example, a swab is used as the specimen collection tool 2 to collect a nasal swab liquid as the specimen 60 by inserting a cotton part at the distal end into the nasal cavity.

Next, the extraction step ST2 of extracting a nucleic acid 64 from the specimen 60 is carried out. In the extraction step ST2, the cotton part to which the specimen 60 of the specimen collection tool 2 is adhered is immersed in the extraction reagent 3. In a case where the specimen 60 is immersed in the extraction reagent 3, the specimen 60 is brought into contact with the extraction reagent 3 containing a nonionic surfactant. The specimen 60 is subjected to mixing in the extraction reagent 3. Cells in the specimen 60 are lysed by the action of the extraction reagent 3, and then the nucleic acid 64 is eluted into the extraction reagent 3. As a result, the nucleic acid extraction liquid 62 is obtained. The nucleic acid extraction liquid 62 contains the impurities 66 that inhibit the amplification of the nucleic acid 64, in addition to the nucleic acid 64. The impurities 66 include impurities derived from a living body, which are contained in the specimen 60, and impurities such as a cell wall, which are generated during the extraction of the nucleic acid.

Next, the purification step ST3 is carried out. The purification step ST3 includes an adsorption step ST31 and a filtration step ST32. First, the zeolite 6 is added to the nucleic acid extraction liquid 62 in the extraction container 4, and the adsorption step ST31 of adsorbing the impurities 66 in the nucleic acid extraction liquid 62 to the zeolite 6 is carried out. In the adsorption step ST31, the zeolite 6 is added to the nucleic acid extraction liquid 62, mixed, and then allowed to stand for a certain time (for example, 10 minutes) to allow the impurities 66 to be adsorbed to the zeolite 6.

Thereafter, in the purification step ST3, the filtration step ST32 of filtering the nucleic acid extraction liquid 62 to remove the zeolite 6 (and the impurities 66 adsorbed to the zeolite 6) in the nucleic acid extraction liquid 62 is carried out. The injection opening 8a of the funnel 8 is allowed to be fitted to the opening portion 4a of the extraction container 4. As a result, the filtration filter 7 provided in the funnel 8 is installed above the nucleic acid extraction liquid 62. The upside of the extraction container 4 is turned down in a state where the funnel 8 has been fitted into the opening portion 4a of the extraction container 4, whereby the nucleic acid extraction liquid 62 is filtered by the filtration filter 7 and then discharged from the discharge opening 8b of the funnel 8. As described above, since the mesh (that is, the pore diameter) of the filtration filter 7 is smaller than the zeolite 6, the zeolite 6 does not pass through the filtration filter 7. As a result, the impurities 66 adsorbed to the zeolite 6 cannot pass through the filtration filter 7. The solution that has not been adsorbed to the zeolite 6 and the nucleic acid 64 smaller than the mesh of the filtration filter 7 pass through the filtration filter 7 and then are discharged from the discharge opening 8b. In the present example, the purified nucleic acid extraction liquid 62 which has been discharged from the discharge opening 8b of the funnel 8 is charged into the cartridge 10 from the inlet 12 of the cartridge 10, which will be described later.

After the purified nucleic acid extraction liquid 62 is charged into the cartridge 10, the cartridge 10 is loaded into the examination device 100, and the amplification step and the examination step are carried out in the examination device 100.

### (Amplification step)

The nucleic acid extraction liquid 62 accommodated in the first accommodating part 16 is heated using the second heating unit 114. The heating makes it possible to inactivate a restriction enzyme, thereby suppressing the decomposition of the extracted nucleic acid. A heating temperature may be a temperature range that does not adversely affect a nucleic acid, and it is preferable that the heating temperature is in a range of, for example, about 50°C to 95°C.

The nucleic acid extraction liquid 62 subjected to heating treatment as pretreatment in the first accommodating part 16 is fed to the second accommodating part 18. The first push rod 101 of the first pressing unit 102 is inserted from the other end 31a open to the outside the first cylinder 31 to press and move the first plug 33 toward the inner space of the cartridge 10. Accordingly, the nucleic acid extraction liquid 62 accommodated in the first accommodating part 16 can be fed to the second accommodating part 18. In this case, the first plug 33 is pushed in and the inner space is pressurized, and thus the second plug 34 positioned in the second cylinder 32 is moved to the outside. Therefore, pressure in the inner space is adjusted and liquid can be fed with weak pressing.

The nucleic acid extraction liquid 62 is fed from the first accommodating part 16 to the second accommodating part 18 through the flow channel 20. The reagent 42 is dissolved since the reagent 42 is provided in the second accommodating part 18 is provided, and the nucleic acid extraction liquid 62 flows into the second accommodating part 18, whereby the nucleic acid extraction liquid 62 is mixed with the reagent 42.

After a mixed liquid of the nucleic acid extraction liquid 62 and the reagent 42 is fed to the second accommodating part 18, the second push rod 103 of the second pressing unit 104 is inserted from the other end 32a open to the outside of the second cylinder 32, presses the second plug 34, and pressurizes the inner space. In this pressurized state, the mixed liquid present in the second accommodating part 18 is heated by the first heating unit 112 and a base sequence as an examination target is amplified. It is noted that although the amplification method is not limited, as an example, a reverse transcription (RT)-PCR method or a PCR method is used. In a case where a PCR method is used, a step of dissociating a double-stranded DNA into a single-stranded DNA at a high temperature (thermal denaturation step), a step of lowering a temperature and binding a primer to the single-stranded DNA (annealing step), and a step of newly synthesizing a double-stranded DNA by polymerase using the single-stranded DNA as a template (elongation step) are repeated. Examples of the temperature cycle for the thermal denaturation step, the annealing step, and the elongation step include a temperature cycle repeated 20 to 50 times, where one cycle is defined as 1 minute at 94°C, 1 minute at 50°C to 60°C, and 1 to 5 minutes at 72°C. In addition, the annealing step and the stretching step may be carried out at one temperature. Examples of such a temperature cycle include a temperature cycle repeated 20 to 50 times, where one cycle is defined as 1 minute at 98°C and 1 minute at 60°C. The temperature and time of the temperature cycle of the amplification step are not particularly limited and are arbitrarily selected depending on the performance of polymerase or a primer.

### (Detection step)

Fluorescence detection is carried out for each cycle of the above-described temperature cycle, and an amplification situation is monitored in real-time. That is, the amplification step and the detection step are carried out in parallel in the present example. Results of the fluorescence detection are displayed on the monitor 130.

In a case where a base sequence as an examination target is present in the nucleic acid extraction liquid 62, this base sequence is amplified in the amplification step, and a fluorescence probe that is labeled to this base sequence as an examination target is irradiated with the excitation light, whereby fluorescence is detected. On the other hand, in a case where a base sequence as an examination target is not present in the nucleic acid extraction liquid 62, fluorescence is not detected even in a case where irradiation is carried out with excitation light. This makes it possible to determine the presence or absence of the base sequence as an examination target.

As described above, the nucleic acid examination method according to the present embodiment includes the extraction step of bringing the specimen 60 into contact with the extraction reagent 3 containing a nonionic surfactant, and the purification step of removing the impurities 66 in the nucleic acid extraction liquid 62. In addition, the purification step includes an adsorption step of bringing any liquid of a liquid containing the specimen 60 or the nucleic acid extraction liquid 62 from which the nucleic acid has been extracted, into contact with the zeolite 6 having any crystal structure of a ferrierite, a mordenite, an L-type, or a Y-type, to adsorb the impurities 66 in the liquid to the zeolite 6.

The inventors of the present invention found that the amplification of the base sequence as an examination target may not be sufficiently realized in a case where, using a method described in WO2009/060847A, the nucleic acid is extracted using an anionic surfactant and then amplified using a PCR method instead of the LAMP method. While the general reaction temperature of the LAMP method is about 65°C, the PCR method generally includes a step of carrying out heating at 90°C or higher. Therefore, in the PCR method, an enzyme having high heat resistance is used as an enzyme to be contained in the amplification reagent. In a case where an anionic surfactant is used, it is presumed that some kind of function such as the activity of an enzyme having high heat resistance, which is used in PCR amplification, is suppressed, whereby the nucleic acid amplification is inhibited. On the other hand, it is possible to suppress the inhibition of the PCR amplification by carrying out the nucleic acid extraction using a nucleic acid extraction liquid containing a nonionic surfactant as in the case of the present embodiment, and thus it is possible to promote the amplification of the base sequence as an examination target and to realize high examination accuracy.

It is noted that the same effect can be obtained by using a nonionic surfactant not only in the PCR amplification but also in an amplification method using an enzyme having high heat resistance, which is not limited to PCR amplification. On the other hand, the nucleic acid examination method according to the present embodiment can also be applied to a case where an amplification method in which an amplification treatment is carried out at a relatively low temperature, such as the LAMP method, is used, where the amplification method is not limited.

In addition, in the adsorption step, the impurities 66 in the liquid (in the example described above, the nucleic acid extraction liquid 62) are adsorbed to the zeolite 6 having the specific crystal structure. Therefore, the impurities 66 adsorbed to the zeolite 6 can be removed from the nucleic acid extraction liquid 62 by removing the zeolite 6 from the nucleic acid extraction liquid 62. Since it is possible to remove the impurities that inhibit the amplification of the base sequence as an examination target in the amplification step, it is possible to promote the amplification of the base sequence as an examination target in the amplification step and to realize high examination accuracy.

As described above, the present nucleic acid examination method includes the extraction step that uses a nonionic surfactant and the purification step of adsorbing impurities to the zeolite 6 having a specific crystal structure to remove the impurities. Then, an amplification step is carried out using a nucleic acid extraction liquid obtained through such an extraction step and purification step. As a result, it is possible to effectively suppress the inhibition of the amplification of the base sequence as an examination target in the amplification step, and it is possible to realize a nucleic acid examination with high examination accuracy as compared with methods in the related art, where the nucleic acid examination is not limited by a specific amplification method.

In the above-described embodiment, a case where the PCR method is used as the amplification method has been described; however, the amplification method is not limited to the PCR method. In addition to the PCR method, the following publicly known amplification method can be used as an amplification method: a LAMP method, a nucleic acid sequence-based amplification (NASBA) method, a transcription reverse-transcription concerted reaction (TRC) method, a recombinase polymerase amplification (RPA) method, a nicking enzyme amplification reaction (NEAR) method, a strand displacement amplification (SDA) method, a helicase-dependent amplification (HDA) method, or the like.

The enzyme to be contained in the amplification reagent that is used in the amplification method includes a reverse transcriptase that synthesizes complementary DNA (cDNA) using RNA as a template and a DNA polymerase that amplifies DNA or cDNA, and it is appropriately selected according to the target to be amplified and the amplification method.

In a case where an RNA virus such as a retrovirus is a target to be amplified, the RNA is converted into cDNA using a reverse transcriptase before the amplification treatment, and then the cDNA is amplified. The cDNA is amplified by an RT-PCR method using the PCR method, an RT-LAMP method using the LAMP method, or the like. On the other hand, in a case where DNA is extracted from a bacterium such as a pathogenic microorganism to amplify the DNA, the DNA is amplified without carrying out reverse transcription.

The reverse transcriptase includes an avian myeloblastosis virus (AMV) reverse transcriptase, a Moloney murine leukemia virus (MMLV) reverse transcriptase, and the like. A mutation is introduced in order to improve the reverse transcription efficiency or extend the chain length of the cDNA to be synthesized. Alternatively, it is also possible to remove the ribonuclease (RNase) H activity.

Examples of the DNA polymerase include thermostable DNA polymerases such as Taq DNA polymerase, Vent DNA polymerase, Deep Vent DNA polymerase, KOD DNA polymerase, TTx DNA polymerase, Tth DNA polymerase, and Pfu DNA polymerase, as well as Bsu DNA polymerase, Bst DNA polymerase, and E. coli DNA polymerase.

In addition, an enzyme that is capable of carrying out reverse transcription and amplification as one enzyme, such as TthDNA polymerase or TTxDNA polymerase, may be used.

Further, enzymes obtained by modifying these enzymes or an enzyme to which an antibody is bound to suppress activity at room temperature or in the reverse transcription step may be used.

As already described, the nucleic acid examination method according to the present disclosure exhibits a remarkable effect in a case where an enzyme having high heat resistance, which is used in the PCR method or the like, is used. As a result, in a case where the PCR method is used in the amplification step, the amplification of the base sequence as an examination target can be promoted, and thus the effect of improving the examination accuracy is high.

In the nucleic acid examination method according to the present embodiment, the purification step includes, after the adsorption step, a filtration step of filtering a liquid (here, the nucleic acid extraction liquid 62) to remove the zeolite from the liquid. The impurities 66 can be easily removed by using filtration as a method of separating the zeolite 6 (and the impurities 66) from the liquid.

It is noted that centrifugal separation or the like can also be used as a method of separating the zeolite 6 from the liquid.

In a case where the extraction reagent 3 contains, as a non-ionic surfactant, at least one of polyethylene oxide, glucamine, or betaine, a high effect of suppressing the amplification inhibition is obtained. In a case where an extraction reagent containing polyethylene oxide among these surfactants is used as a nonionic surfactant, the highest effect of suppressing the amplification inhibition is obtained (see Examples shown in Table 1 later).

In a case where the zeolite 6 has any crystal structure of a ferrierite, an L-type, or a Y-type crystal structure, the effect of removing impurities is high as compared with a case where zeolite having a crystal a mordenite crystal structure is used (see Examples shown in Table 1 later).

In a case where the cation of the zeolite 6 is any one of Na⁺, K⁺, or H⁺, the adsorptivity of the impurities 66 is high as compared with a case where HN₄⁺ is used as a cation. As a result, the effect of removing the impurities 66 is high, and thus the effect of suppressing the amplification inhibition is high (see Examples shown in Table 1 later).

In a case where the micropore diameter of the zeolite 6 is more than 0.7 nm and less than 1 nm, high adsorptivity for adsorbing the impurities 66 can be obtained. (See Examples shown in Table 1 later).

In a case where the ratio Si/Al of silicon to aluminum in the zeolite 6 is 5 or more and 18 or less, the adsorptivity of the impurities 66 is high. As a result, the effect of removing the impurities 66 is high, and thus the effect of suppressing the amplification inhibition is high (see Examples shown in Table 1 later).

The examination kit 1 according to the above-described embodiment includes the extraction container 4 accommodating the extraction reagent 3 that contains a nonionic surfactant, the zeolite 6 having any crystal structure of a ferrierite, a mordenite, an L-type, or a Y-type, and the cartridge 10. In addition, the cartridge 10 includes a plurality of the accommodating parts 16 and 18 including an amplification reagent accommodating part (here, the second accommodating part 18) that accommodates an amplification reagent (here, the reagent 42 including the amplification reagent) to be used for amplifying the nucleic acid 64; and a plurality of the flow channels 20, 24, and 26 including the flow channel 20 that connects between a plurality of the accommodating parts 16 and 18. In a case where the examination kit 1 of the present configuration is used, the nucleic acid examination method according to the present disclosure can be easily realized.

In the examination kit 1, the zeolite 6 is provided in an aspect in which the zeolite 6 is capable of being charged into the extraction container 4. However, it is sufficient that the zeolite 6 is provided in at least one of the extraction container 4 or the cartridge 10.

The examination kit 1 according to the above-described embodiment includes a filter for filtering the nucleic acid extraction liquid 62, the filter being the filtration filter 7 having meshes smaller than the zeolite 6. In a case where such a filtration filter 7 is included, the impurities 66 adsorbed to the zeolite 6 can be efficiently separated from the nucleic acid extraction liquid 62.

In the examination kit 1, the filtration filter 7 is provided separately from the cartridge 10, and specifically, the filtration filter 7 is provided in the funnel 8. It suffices that the filtration filter 7 is provided in a path through which the nucleic acid extraction liquid 62 which is a liquid in the extraction container 4 is charged into the cartridge 10, and the aspect of the filtration filter 7 is not limited to an aspect in which the filtration filter 7 is provided in the funnel 8. In addition, the filtration filter 7 may be provided in the cartridge 10. For example, the filtration filter 7 may be configured such that the filtration filter 7 is provided at the inlet 12 of the cartridge 10 and the nucleic acid extraction liquid 62 filtered at the inlet 12 is supplied to the first accommodating part 16 of the cartridge 10.

Since the examination kit 1 includes the funnel 8, the nucleic acid extraction liquid 62 can be easily charged into the cartridge 10 from the extraction container 4. However, the examination kit 1 may not include the funnel 8. In a case where the examination kit 1 does not include the funnel 8, the nucleic acid extraction liquid 62 may be sucked from the extraction container 4 using a pipette or the like, and the nucleic acid extraction liquid 62 may be charged into the cartridge 10 from the inlet 12.

In the nucleic acid examination method according to the above-described embodiment, the purification step is carried out after the extraction step as shown in Fig. 6 and Fig. 7. However, as described above, the purification step may be carried out at any stage before, during, or after the extraction step.

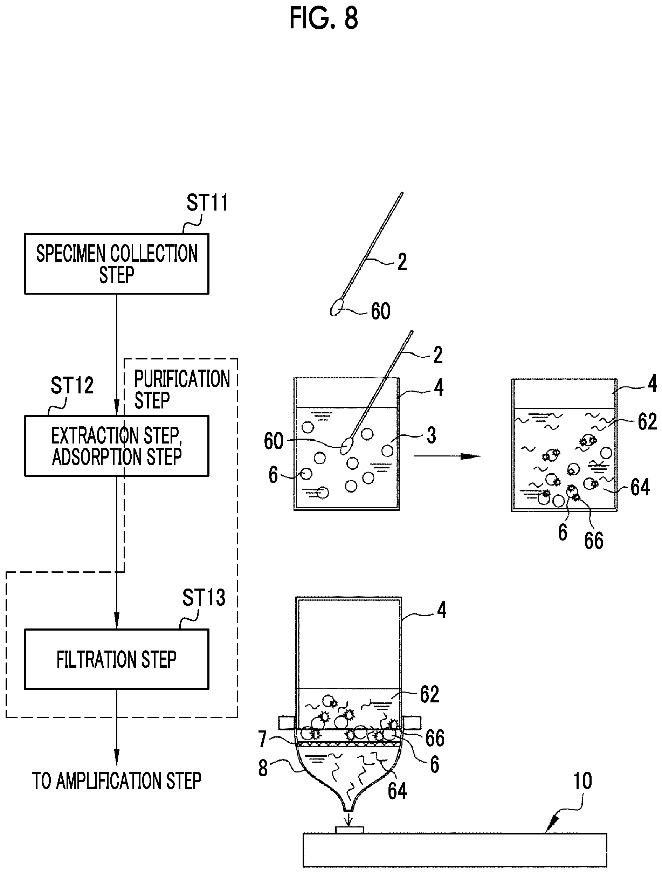

For example, as shown in Fig. 8, the extraction step and the adsorption step may be carried out in parallel (a step ST12) after a collection step ST11, and then a filtration step ST13 may be carried out.

In this case, the zeolite 6 is added in advance to the extraction reagent 3 in the extraction container 4. The cotton part to which the specimen 60 of the specimen collection tool 2 has been adhered is immersed in the extraction reagent 3 to which the zeolite 6 has been added. As a result, the specimen 60 is eluted into the extraction reagent 3, and cells are lysed by the action of the extraction reagent 3 to elute the nucleic acid 64. Since the zeolite 6 is added to the extraction reagent 3, the impurities 66 included in the specimen 60 and the impurities 66 generated during the cell lysis are adsorbed to the zeolite 6 in parallel with the extraction step.

Thereafter, the purified nucleic acid extraction liquid 62 is charged into the cartridge 10 through the filtration step ST13.

By carrying out the extraction step and the adsorption step in parallel in the step ST12, the time required for the extraction step and the purification step can be shortened as compared with a case where the purification step is carried out after the extraction step.

It is noted that, in this way, in a case where the extraction step and the adsorption step are carried out in parallel, the zeolite 6 may be provided in advance in the extraction container 4 in the examination kit 1.

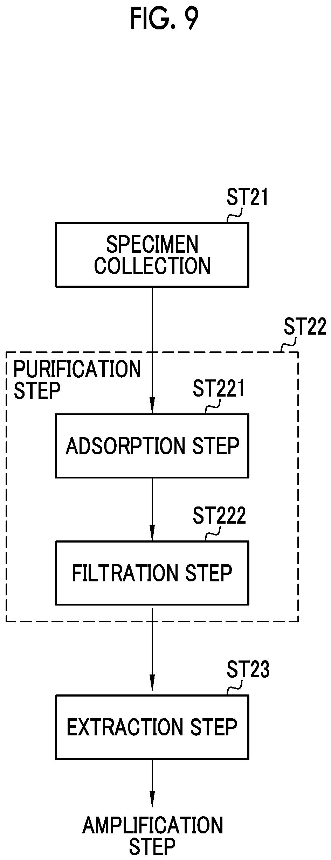

In addition, as shown in Fig. 9, a purification step ST22 may be carried out after a collection step ST21 and before carrying out an extraction step ST23.

In this case, in the purification step ST22, the adsorption step ST221 and the filtration step ST222 are carried out in this order.

In the adsorption step ST221, the specimen 60 collected with the specimen collection tool 2 is eluted into a solution such as a buffer solution, and the zeolite 6 is added to a buffer solution containing the specimen 60. The impurities 66 included in the specimen 60 is adsorbed to the zeolite 6 in the buffer solution.

In the filtration step ST222, the buffer solution after the adsorption step is filtered using the filtration filter 7.

Thereafter, as the extraction step ST23, the extraction reagent 3 is added to the filtered buffer solution to extract a nucleic acid.

In this way, in a case where the purification step ST22 is carried out before the extraction step ST23, the impurities 66 that can be adsorbed and removed by the zeolite 6 are impurities derived from a living body. Since the extraction step ST23 is not yet carried out, impurities generated during the extraction of the nucleic acid are not generated in the adsorption step ST22. Therefore, the nucleic acid extraction liquid 62 obtained after the extraction step ST23 contains impurities generated during the extraction of the nucleic acid, and the amplification step is carried out in a state where such impurities are included. However, in the amplification step, the main impurities 66 that inhibit the amplification of the nucleic acid 64 are impurities derived from a living body, Therefore, an effect of suppressing the amplification inhibition can be obtained even in a case where the purification step ST22 is carried out before the extraction step ST23.

However, as shown in Figs. 6 to 9, the purification step is preferably carried out after the extraction step or in the middle of the extraction step. In the purification step, the impurities 66 generated in the extraction step can also be removed, and thus the effect of suppressing the amplification inhibition is high.

### "Modification example of cartridge"

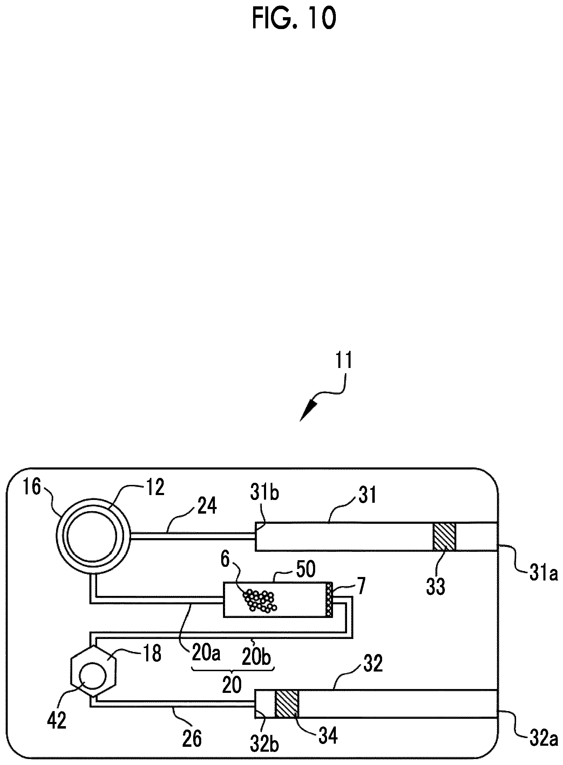

Fig. 10 is a plan view schematically showing a cartridge 11 of a modification example. The same reference numerals are assigned to constitutional elements that are the same as those of the cartridge 10 shown in Fig. 1, and detailed descriptions thereof will be omitted.

The cartridge 11 includes a purification chamber 50 in the middle of the first flow channel 20 that connects the first accommodating part 16 and the second accommodating part 18. The first flow channel 20 is composed of a first flow channel-first portion 20a that connects the first accommodating part 16 to the purification chamber 50, and a first flow channel-second portion 20b that connects the purification chamber 50 to the second accommodating part 18.

The purification chamber 50 is a chamber in which the purification step of removing the impurities 66 from the nucleic acid extraction liquid 62 is carried out. In the present example, the zeolite 6 is provided in the purification chamber 50. That is, the zeolite 6 is provided in the cartridge 11. In addition, The filtration filter 7 is provided at a downstream end of the purification chamber 50 that is connected to the first flow channel-second portion 20b.

In the examination kit 1 shown in Fig. 1, the funnel 8 includes the filtration filter 7; however, in the present example, the filtration filter 7 is provided in the purification chamber 50. In a case where the present cartridge 11 is used, the funnel 8 does not need the filter 7.

In a case where the cartridge 10 shown in Fig. 1 is used, the purification step is carried out before the nucleic acid extraction liquid 62 is added to the cartridge 10, and the purified nucleic acid extraction liquid 62 is charged into the cartridge 10. On the other hand, in a case where an examination kit including the present cartridge 11 is used, only the extraction step is carried out outside the cartridge 11, and the purification step, the amplification step, and the detection step are carried out inside the cartridge 11.

The unpurified nucleic acid extraction liquid 62 is charged into the cartridge 11, and the nucleic acid extraction liquid 62 is fed to the purification chamber 50 from the first accommodating part 16 through the first flow channel first part 20a. In the purification chamber 50, the nucleic acid extraction liquid 62 is mixed with the zeolite 6, and the impurities 66 in the nucleic acid extraction liquid 62 are adsorbed to the zeolite 6. Thereafter, the nucleic acid extraction liquid 62 is fed to the second accommodating part 18 through the filtration filter 7 and the first flow channel-second portion 20b. The nucleic acid extraction liquid 62 is filtered by the filtration filter 7, the zeolite 6 and the impurities 66 adsorbed to the zeolite 6 are left in the purification chamber 50, and the purified nucleic acid extraction liquid 62 is fed to the second accommodating part 18. The subsequent amplification step and detection step are the same as those in the case of using the cartridge 10.

Even in a case where the cartridge 11 of the modification example is used, it is possible to carry out a nucleic acid examination method which includes the extraction step that uses a nonionic surfactant and the purification step of adsorbing impurities to the zeolite 6 having a specific crystal structure to remove the impurities. As a result, it is possible to effectively suppress the inhibition of the amplification of the base sequence as an examination target in the amplification step, and it is possible to realize a nucleic acid examination with high examination accuracy as compared with methods in the related art, where the nucleic acid examination is not limited by a specific amplification method.

As described above, the cartridge 11 includes the purification chamber 50, and the zeolite 6 and the filtration filter 7 are provided in the cartridge 11. Specifically, the cartridge 11 includes the purification chamber 50 as one accommodating part different from the second accommodating part 18 which is the amplification reagent accommodating part, and the zeolite 6 is accommodated in the purification chamber 50. In addition, the filtration filter 7 is provided at an end part of the purification chamber 50, the end part being in a flow channel from the purification chamber 50 to the second accommodating part 18.

According to the cartridge 11 of the modification example, the purification step can be carried out in the cartridge 11. As a result, A user who carries out an examination can reduce the burden by reducing work carried out by manual operations.

### Examples

Hereinafter, more specific Examples and Comparative Examples of the technology according to the present disclosure will be described.

In Examples and Comparative Examples, a change was made in a combination of a surfactant, which is contained in a reagent for extracting a nucleic acid which is used in the nucleic acid extraction step, and an adsorbent which adsorbs impurities in the purification step. For Examples and Comparative Examples shown in Table 1 and Table 2, the adsorptivity of the impurities, the adsorptivity of the nucleic acid, and the amplification rate in a case where PCR amplification was carried out were evaluated.

In Examples 1 to 21, a nonionic surfactant was used as the surfactant. In addition, in Examples 1 to 21, a zeolite having any structure of a ferrierite, a mordenite, an L-type, or a Y-type was used as the adsorbent.

In Examples 1 to 7, the crystal structure of the zeolite and the cation species were changed.

Examples 8 to 11 of the zeolite in Table 1 are examples in which the concentration of the zeolite was changed with respect to Example 2. Examples 12 to 18 are examples in which the kind of the surfactant was changed with respect to Example 2, and Examples 19 to 21 are examples in which the concentration of the surfactant was changed.

Comparative Examples 1 to 3 shown in Table 2 are examples in which an anionic surfactant was used as the surfactant. In Comparative Examples 4 and 5, the structure of the zeolite is different from those of Examples. In addition, Comparative Examples 6 to 14 are examples in which an adsorbent other than the zeolite was used. In Comparative Examples 6 to 8, activated carbon was used as the adsorbent, and the characteristics of each activated carbon are described in the item of the product number of the activated carbon. Specifically, activated carbon, greener alternative DARCO (registered trademark) manufactured by Sigma-Aldrich Co. LLC, 12-20 mesh, granular, was used in Comparative Example 6, activated carbon (powder, neutral) manufactured by FUJIFILM Wako Pure Chemical Corporation was used in Comparative Example 7, and activated carbon (powder, alkaline) manufactured by FUJIFILM Wako Pure Chemical Corporation was used in Comparative Example 8.

In Table 1, the micropore diameter [× 10⁻¹ nm], the Si/Al ratio, and the specific surface area [m²/g] of the zeolite are all nominal values according to the manufacturer. "Undisclosed" in Table 1 means that the information is not disclosed by the manufacturer of each adsorbent.

### "Evaluation of adsorptivity"

### (Evaluation of adsorptivity of impurities by adsorbent)

Lysozyme was used as the impurities present in the nasal cavity, and the adsorptivity of lysozyme by the adsorbent was evaluated. For an aqueous solution containing the lysozyme as impurities, a ratio of the concentration of the lysozyme before and after carrying out the purification using the adsorbent was determined as an adsorption rate of the lysozyme by the adsorbent.

### - Preparation of lysozyme aqueous solution -

First, a lysozyme aqueous solution having the following composition was prepared. Lysine and deionized distilled water were weighed and then subjected to stirring with a vortex mixer to prepare a lysozyme aqueous solution.

### -- Composition of lysozyme aqueous solution --

Lysozyme, egg white-derived (manufactured by Wako Pure Chemical Corporation): 0.50 g
Deionized distilled water
(manufactured by NIPPON GENE CO., LTD., RNase/DNase free): 99.5 g

### - Preparation of adsorbent dispersion liquid -

Zeolite and water were weighed and then stirred with a vortex mixer to prepare an adsorbent dispersion liquid. The composition of a zeolite dispersion liquid 1, which is the adsorbent dispersion liquid of Example 1, is as follows. In each of Examples and Comparative Examples, the total amount of the zeolite or another adsorbent (activated carbon, a styrene-based polymer, or a polymethacrylate) and the deionized distilled water was set to 0.85 g, and each of adsorbent dispersion liquids were prepared so that the concentration [wt/vol%] of the adsorbent in the mixed liquid described below was the value shown in Table 1.

### -- Composition of zeolite dispersion liquid 1 --

Zeolite HS-320 (manufactured by Wako Pure Chemical Corporation): 0.075 g
Deionized distilled water
(manufactured by NIPPON GENE CO., LTD., RNase/DNase free): 0.775 g

### - Preparation of adsorbent-lysozyme mixed liquid -

Next, the lysozyme aqueous solution and the adsorbent dispersion liquid were mixed to prepare an adsorbent-lysozyme mixed liquid. The above-described lysozyme aqueous solution was added to the adsorbent dispersion liquid, and then the resultant mixture was stirred with a vortex mixer to prepare an adsorbent-lysozyme mixed liquid. In the case of Example 1, the zeolite dispersion liquid 1 and the lysozyme aqueous solution were mixed at the following weight ratio to prepare a zeolite-lysozyme mixed liquid 1. In each of the other Examples and Comparative Examples, the weight ratio of each adsorbent dispersion liquid to each lysozyme aqueous solution was set to be the same as the weight ratio of the zeolite dispersion liquid to the lysozyme aqueous solution in the zeolite-lysozyme mixed liquid 1.

### -- Zeolite-lysozyme mixed liquid 1 --

Zeolite dispersion liquid 1: 0.85 g
Lysozyme aqueous solution: 0.15 g

In the manner as described above, a mixed liquid of the lysozyme aqueous solution and the adsorbent dispersion liquid was prepared, and then the mixed liquid was allowed to stand at room temperature for 10 minutes. Thereafter, the mixed liquid was added into a Terumo Syringe (registered trademark) lock base (manufactured by Terumo Corporation, capacity: 2.5 mL), in which a syringe filter made of polyethersulphone (PES) having a pore size of 0.45 µm (manufactured by GVS Filter Technology) was set, and a filtration treatment was carried out by manual operation.

Using Qubit Protein Assays (manufactured by Thermo Fisher Scientific, Inc.), the concentrations of the lysozyme before and after the purification treatment of the adsorbent-lysozyme mixed liquid were measured. Specifically, a lysozyme concentration D0 in the adsorbent-lysozyme mixed liquid before addition to the syringe was measured, and a lysozyme concentration D1 in the solution after filtering the adsorbent-lysozyme mixed liquid to carry out the purification treatment was measured. Then, (D0 - D1)/D0 was calculated as the adsorption rate.

The determination of the adsorptivity was carried out according to the following standards.
A: Adsorption rate is 80% or more.
B: Adsorption rate is 50% or more and less than 80%.
C: Adsorption rate is 20% or more and less than 50%.
D: Less than 20%

In terms of practical use, an adsorbent having an adsorptivity of C or more is required.

### (Evaluation of adsorptivity of nucleic acid by adsorbent)

Using the nucleic acid extracted from the influenza virus, the adsorptivity of the nucleic acid to the adsorbent was evaluated by the following procedure.

The ratio of the copy number of the nucleic acid before and after the zeolite purification was calculated according to the calibration curve method using a real-time PCR device (CFX96, manufactured by Bio-Rad Laboratories, Inc.) and used as a permeation rate. It is meant that the higher the permeation rate, the lower the adsorption rate. It is desirable to be a rate (permeation rate) at which the nucleic acid is not adsorbed to the adsorbent but is allowed to pass through a filter in the filtration step of the purification treatment.

First, a nucleic acid aqueous solution, in which AmpliRun Influenza AH1 RNA Control (manufactured by Vircell, S.L.) was diluted to 250 Copy/µL, was prepared.

Next, the adsorbent and the nucleic acid aqueous solution were weighed so that the concentration of the adsorbent in the nucleic acid aqueous solution was a value of the concentration of the adsorbent shown in Table 1, and then the adsorbent was dispersed in the nucleic acid aqueous solution with a vortex mixer. Thereafter, the nucleic acid aqueous solution in which the adsorbent was dispersed was allowed to stand at room temperature for 10 minutes. Further, thereafter, this solution was added into a Terumo Syringe (registered trademark) lock base (manufactured by Terumo Corporation, capacity: 2.5 mL) in which a syringe filter made of PES having a pore diameter of 0.45 µm (manufactured by GVS Filter Technology) was set, and a filtration treatment was carried out by manual operation.

APCR reaction solution having the following composition was prepared, and the nucleic acid aqueous solution purified according to the above-described procedure was added thereto to carry out RT-PCR.

In addition, for the present evaluation, RT-PCR was carried out using AmpliRun Influenza A H1 RNA Control (manufactured by Vircell, S.L.) at the following concentration to create a calibration curve of the number of copies of nucleic acid and the Ct value. Specifically, a nucleic acid aqueous solution having a concentration of each of 1,000 Copy/µL, 500 Copy/µL, 250 Copy/µL, 100 Copy/µL, 50 Copy/µL, 25 Copy/µL, 10 Copy/µL, and 5 Copy/µL was prepared. RT-PCR was carried out for the nucleic acid aqueous solution having each of the concentrations, and a Ct value was measured. Based on this result, a calibration curve showing a relationship between the Ct value and the concentration (that is, the number of copies of the nucleic acid) was created.

### - Composition of PCR reaction solution -

One Step PrimeScript RT-qPCR Mix (2x) (manufactured by Takara Bio Inc.)

| | |
|---|---|
| | 10 µL |
| 10 µM forward primer | 1.2 µL |
| 10 µM reverse primer | 1.2 µL |

| | |
|---|---|
| 5 µM probe | 0.4 µL |
| Nucleic acid aqueous solution | 1.0 µL |
| Deionized distilled water (manufactured by NIPPON GENE CO., LTD., RNase/DNase free) | 6.2 µL |
| Total | 20 µL |

As the primer and the probe, the following primers and probe having the following sequences, which have been open to the public respectively as MP-39-67For, MP-183-153Rev, and MP-96-75ProbeAs, were synthesized with reference to the sequences for A type identification published from National Institute of Infectious Diseases (the sequences disclosed in the influenza diagnostic manual published by National Institute of Infectious Diseases).
· Forward primer: CCMAGGTCGAAACGTAYGTTCTCTCTATC (SEQ ID NO: 1)
· Reverse primer: TGACAGRATYGGTCTTGTCTTTAGCCAYTCCA (SEQ ID NO: 2)
· Probe: ATYTCGGCTTTGAGGGGGCCTG (SEQ ID NO: 3)

A probe in which 5'-fluorescein CE-phosphoramidite (FAM) was bound to the 5' terminal side of the above-described probe sequence (SEQ ID NO: 3), and a probe in which a minor groove binder (MGB) and a non-fluorescent quencher (NFQ) were bound to the 3' terminal side thereof were used as the probe.

The conditions for the RT-PCR were as follows.
Reverse transcription: 50°C, 2 minutes → 95°C, 5 minutes
PCR: 50 cycles of 95°C, 5 seconds ←→ 56°C, 10 seconds

The adsorbent was added to a nucleic acid aqueous solution containing 250 Copy of the nucleic acid, and then the above-described RT-PCR was carried out using a purified nucleic acid aqueous solution obtained by purification, whereby a Ct value was measured. The obtained Ct value was compared with those of the calibration curve described above to determine the number C1 of copies of the nucleic acid in the purified nucleic acid aqueous solution. The number of copies C0 of the nucleic acid present in the PCR reaction field before purification is 250 Copy. A ratio C1/C0 of the numbers of copies of the nucleic acid before and after the purification was used as the permeation rate.

The nucleic acid adsorptivity to the adsorbent was evaluated according to the following standards.
A: Permeation rate is 70% or more.
B: Permeation rate is 20% or more and less than 70%.
C: Permeation rate is less than 20%, or no amplification is observed and thus the Ct value is not calculated with the nucleic acid after the purification.

In terms of practical use, a performance of B or higher is required.

### "Amplifiability evaluation"

A specimen simulating liquid containing each of lysozyme as impurities present in the nasal cavity, a nucleic acid extracted from Influenza as a specimen, and a surfactant was prepared, and the amplifiability of the nucleic acid was evaluated using a real-time PCR device (CFX96, manufactured by Bio-Rad Laboratories, Inc.) according to the following procedure.

### - Preparation of specimen simulating liquid -

For each of Examples and Comparative Examples, a specimen simulating liquid containing each of the surfactants shown in Table 1 or Table 2 was prepared. The composition of a specimen simulating liquid 1 used in Example 1 is as follows. In the case of Example 1, the specimen simulating liquid 1 was prepared by stirring each of the following solutions with a vortex mixer. In Examples 2 to 11 and Comparative Examples 4 to 14, the same specimen simulating liquid 1 as in Example 1 was used. It is noted that in Examples 12 to 21 and Comparative Examples 1 to 3, the components other than the surfactant were equivalent to those of the specimen simulating liquid 1, the surfactant was replaced with the surfactant shown in Table 1, and preparations were made such that the surfactant concentration [vol%] shown in Table 1 was obtained.

### -- Composition of specimen simulating liquid 1 --

### Nucleic acid: AmpliRun Influenza A H1 RNA Control (1,000 Copy/µL)

| | |
|---|---|
| | 12.0 µL |
| Lysozyme (1% solution diluted with water) | 10.2 µL |
| Surfactant: Tween 20 (5% solution diluted with water) (Concentration at purification: 0.5 vol%) | 24.2 µL |
| Water | 195.6 µL |

Next, the adsorbent (zeolite in Example 1) shown in Table 1 and the specimen simulating liquid were weighed to have a concentration [wt/vol%] shown in Table 1, and the resultant mixture was stirred with a vortex mixer. In the case of Example 1, 0.0073 g of the zeolite was weighed, 96.8 µL of the specimen simulating liquid was added thereto, and the resultant mixture was stirred with a vortex mixer. In Example 1, the concentration was 0.0073 g/0.0968 ml ≈ 7.5 [wt/vol%]. After stirring with a vortex mixer, the mixture was allowed to stand at room temperature for 10 minutes. Thereafter, the specimen simulating liquid in which the adsorbent was dispersed was added into a Terumo Syringe (registered trademark) lock base (manufactured by Terumo Corporation, capacity: 2.5 mL) in which a syringe filter made of PES having a pore diameter of 0.45 µm (manufactured by GVS Filter Technology) was set, and a filtration treatment was carried out by manual operation.

Next, a PCR reaction solution having the following composition was prepared, and RT-PCR was carried out using the specimen simulating liquid which had been subjected to the above-described purification treatment.

### -- Composition of PCR reaction solution --

### One Step PrimeScript RT-qPCR Mix (2×)

| | |
|---|---|
| (manufactured by Takara Bio Inc.) | 10 µL |
| 10 µM forward primer | 1.2 µL |
| 10 µM reverse primer | 1.2 µL |
| 5 µM probe | 0.4 µL |
| Specimen simulating liquid | 7.2 µL |
| Total | 20 µL |

Similarly to the case of the evaluation of the adsorptivity of nucleic acid, as the primer and the probe, the following primers and probe having the following sequences, which have been open to the public respectively as MP-39-67For, MP-183-153Rev, and MP-96-75ProbeAs, were synthesized with reference to the sequences for A type identification published from National Institute of Infectious Diseases.
· Forward primer: CCMAGGTCGAAACGTAYGTTCTCTCTATC (SEQ ID NO: 1)
· Reverse primer: TGACAGRATYGGTCTTGTCTTTAGCCAYTCCA (SEQ ID NO: 2)
· Probe: ATYTCGGCTTTGAGGGGGCCTG (SEQ ID NO: 3)

A probe in which 5'-fluorescein CE-phosphoramidite (FAM) was bound to the 5' terminal side of the above-described probe sequence (SEQ ID NO: 3), and a probe in which a minor groove binder (MGB) and a non-fluorescent quencher (NFQ) were bound to the 3' terminal side thereof were used as the probe.

The conditions for the RT-PCR were as follows.
Reverse transcription: 60°C, 1 minute → 95°C, 1 minute
PCR: 50 cycles of 95°C for 1 second ←→ 60°C for 6 seconds

For a Ct value obtained after the RT-PCR, the Ct value being a value with respect to the amplification curve, the determination was made according to the following standards.
4: Ct value is 35 or less.
3: Ct value is more than 35 and 37 or less.
2: Ct value is more than 37 and 40 or less.
1: More than 40.

The smaller the Ct value is, the more preferable it is. In terms of practical use, a performance of 40 or less (determination: 2 or higher) as the Ct value is required.

Table 1 summarizes the conditions of the surfactant and the adsorbent and the evaluation results for Examples. In addition, Table 2 summarizes the conditions of the surfactant and the adsorbent and the evaluation results for Comparative Examples.

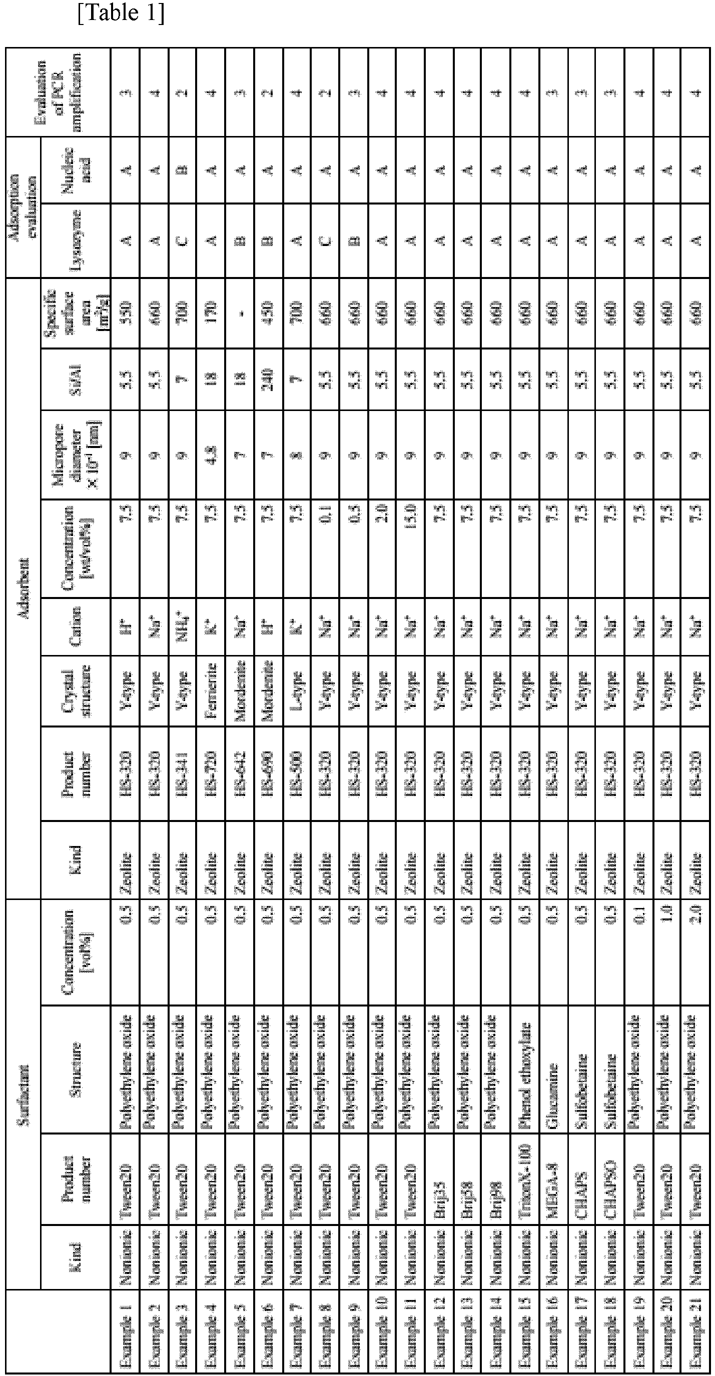

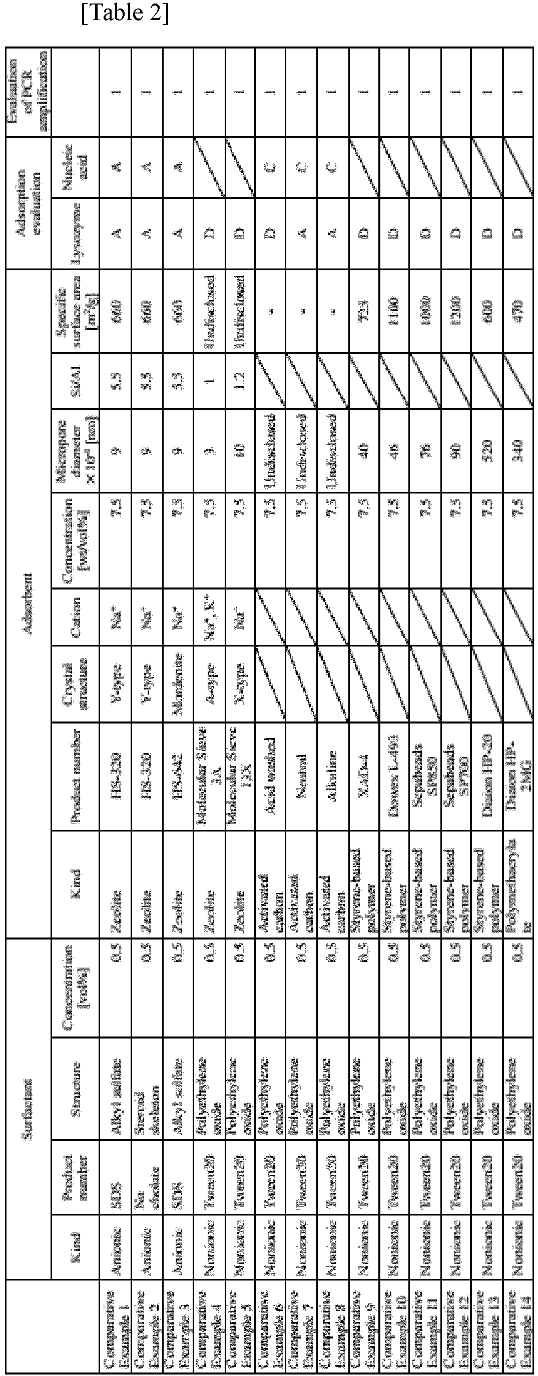

As shown in Table 1 and Table 2, Examples 1 to 21 are examples in which an extraction reagent containing a nonionic surfactant was used and a zeolite having any crystal structure of a ferrierite, a mordenite, an L-type, or a Y-type was used. In Examples 1 to 21, the obtained results were such that the adsorptivity of the impurities (here, the lysozyme) is as high as C or higher, the permeation rate of the nucleic acid is as high as B or higher, that is, the adsorption rate of the nucleic acid is low, and the amplifiability of the nucleic acid is as high as a level 2 or higher, and thus the practical performance is satisfied.

In Comparative Examples 1 to 3 in which an extraction reagent containing an anionic surfactant was used, although good results were obtained in the adsorption evaluation, the amplifiability of the nucleic acid was low. This is considered to be a result indicating that the anionic surfactant inhibits the amplification of the nucleic acid.

In any of Comparative Examples 4 to 14 in which an A-type or X-type zeolite, or an adsorbent which is not the zeolite was although an extraction reagent containing a nonionic surfactant was used, the adsorptivity of the impurities was low, or the nucleic acid was adsorbed. As a result, the amplifiability was low.

According to the evaluation results of Examples 1 to 3 in which only the cation species of the zeolite was changed, H⁺ or Na⁺ gives good adsorptivity as a cation species as compared with NH₄⁺.

According to the evaluation results of Example 2 and Examples 4 to 7 in which only the crystal structure of the zeolite was different, the ferrierite, the L-type, and the Y-type give good adsorptivity of impurities as compared with the mordenite.

According to the evaluation results of Example 2 and Examples 8 to 11 in which only the concentration of the zeolite added to the nucleic acid extraction liquid (the simulated sample solution in Examples) was different, the effect of the adsorptivity of the impurities was obtained in a case where the concentration of the zeolite was at least in a range of 0.1 wt/vol% to 15.0 wt/vol%. From the viewpoint of the adsorptivity, the concentration of the zeolite is preferably 0.5 wt/vol% or more and more preferably 2 wt/vol% or more.

According to the evaluation results of Example 2 and Examples 12 to 18 in which only the kind (or product number) of the nonionic surfactant to be used in the extraction reagent is different, polyethylene oxide is preferable since it has a high effect of suppressing amplifiability inhibition as compared with other nonionic surfactants.

According to the evaluation results of Example 2 and Examples 19 to 21 in which only the surfactant concentration in the nucleic acid extraction liquid (the simulated sample solution in Examples) was different, substantially the same effect was obtained in a case where the surfactant concentration was in a range of 0.5 vol% to 2.0 vol%.

The disclosure of JP2021-191592 filed on November 25, 2021, is incorporated in the present specification by reference in its entirety.

All documents, patent applications, and technical standards described in the present specification are incorporated herein by reference, to the same extent as in the case where each of the documents, patent applications, and technical standards is specifically and individually described.

### [Sequence list]

International application based on the International Patent Cooperation Treaty
21F01235WIJP22042617_2.xml

## Claims

1. A nucleic acid examination method for examining whether or not a base sequence as an examination target is present in a nucleic acid contained in a specimen collected from a living body, the nucleic acid examination method comprising:
an extraction step of extracting the nucleic acid from the specimen;
a purification step of removing impurities in a liquid of any of a solution containing the specimen or a nucleic acid extraction liquid from which the nucleic acid has been extracted, at any stage before, during, or after the extraction step;
an amplification step of amplifying the base sequence in the nucleic acid extraction liquid; and
a detection step of detecting presence or absence of the base sequence after the amplification step,
wherein the extraction step is a step of bringing the specimen into contact with an extraction reagent containing a nonionic surfactant, and
wherein the purification step includes an adsorption step of bringing the liquid into contact with a zeolite having any crystal structure of a ferrierite, a mordenite, an L-type, or a Y-type, to adsorb the impurities in the liquid to the zeolite.

2. The nucleic acid examination method according to claim 1,
wherein the purification step includes, after the adsorption step, a filtration step of filtering the liquid to remove the zeolite from the liquid.

3. The nucleic acid examination method according to claim 1 or 2,
wherein, in the extraction step, the zeolite is added into the extraction reagent to carry out the extraction step and the purification step in parallel.

4. The nucleic acid examination method according to claim 1 or 2,
wherein the purification step is carried out after the extraction step.

5. The nucleic acid examination method according to any one of claims 1 to 4,
wherein the nonionic surfactant includes at least one of polyethylene oxide, glucamine, or betaine.

6. The nucleic acid examination method according to any one of claims 1 to 5,
wherein the zeolite has any crystal structure of a ferrierite, an L-type, or a Y-type.

7. The nucleic acid examination method according to any one of claims 1 to 6,
wherein a cation of the zeolite is any one of Na⁺, K⁺, or H⁺.

8. The nucleic acid examination method according to any one of claims 1 to 5,
wherein a micropore diameter of the zeolite is more than 0.7 nm and less than 1 nm.

9. The nucleic acid examination method according to any one of claims 1 to 8,
wherein a ratio Si/Al of silicon to aluminum in the zeolite is 5 or more and 18 or less.

10. The nucleic acid examination method according to any one of claims 1 to 9,
wherein the amplification step is an amplification step by a polymerase chain reaction.

11. An examination kit that is used for examining whether or not a base sequence as an examination target is present in a nucleic acid contained in a specimen collected from a living body, the examination kit comprising:
an extraction container accommodating an extraction reagent that contains a nonionic surfactant; and
a cartridge including:
a plurality of accommodating parts that includes an amplification reagent accommodating part accommodating an amplification reagent that is used for amplification of the nucleic acid, and
a plurality of flow channels that connect the plurality of accommodating parts to each other,
wherein a zeolite having any crystal structure of a ferrierite, a mordenite, an L-type, or a Y-type is provided in at least one of the extraction container or the cartridge or in a form in which the zeolite is capable of being charged into the extraction container.

12. The examination kit according to claim 11,
wherein a filter having meshes smaller than the zeolite is provided, the filter being used for filtering a nucleic acid extraction liquid.

13. The examination kit according to claim 12,
wherein the filter is provided in the cartridge or separately from the cartridge.

14. The examination kit according to claim 13,
wherein the filter is provided in a path through which a liquid in the extraction container is charged into the cartridge or at an inlet of the cartridge for the liquid.

15. The examination kit according to claim 13,
wherein the cartridge accommodates the zeolite in one accommodating part that is different from the amplification reagent accommodating part among the plurality of accommodating parts, and
wherein the cartridge includes the filter in a flow channel from the accommodating part in which the zeolite is accommodated to the amplification reagent accommodating part.

16. The examination kit according to any one of claims 11 to 15,
wherein the cartridge includes;
an inlet into which a liquid is to be charged,
an attachable and detachable lid portion that covers the inlet,
a first accommodating part that is capable of accommodating a liquid and is provided at a position facing the inlet,
a second accommodating part that is the amplification reagent accommodating part accommodating the amplification reagent,
a first flow channel that connects the first accommodating part to the second accommodating part,
a first cylinder in which one end is connected to the first accommodating part through a second flow channel and another end is open to an outside,
a second cylinder in which one end is connected to the second accommodating part through a third flow channel and another end is open to the outside,
a first plug that is provided to be movable in the first cylinder, and
a second plug that is provided to be movable in the second cylinder,
wherein the cartridge is configured such that an inner space including the first accommodating part, the second accommodating part, the first flow channel, the second flow channel, and the third flow channel is capable of being pressurized by pressing the first plug and the second plug to be moved from the outside,
wherein each of the first accommodating part and the second accommodating part is one of the plurality of accommodating parts, and
wherein each of the first flow channel, the second flow channel, and the third flow channel is one of the plurality of flow channels.
